# EUROPEAN PATENT SPECIFICATION

(11) **EP 1 070 067 B1**
(45) Date of publication and mention of the grant of the patent: **02.03.2005**
(21) Application number: 98962610.6
(22) Date of filing: 23.12.1998
(51) Int. Cl.: C07D 487/06, A61K 31/505

(54) **CYTOTOXIC ALKALOID DERIVATIVES INCLUDING ASMARINE A AND B ISOLATED FROM A SPONGE**
ZYTOTOXISCHE ALKALOIDDERIVATE, DIE ASMARIN A UND B UMFASSEN, UND AUS EINEM SCHWAMM ISOLIERT WURDEN
DERIVES D'ALCALOIDES CYTOTOXIQUES TELS QUE L'ASMARINE A ET B ISOLES A PARTIR D'UNE EPONGE

(30) Priority: 23.12.1997 GB 9727301
(43) Date of publication of application: 24.01.2001
(73) Proprietor: Instituto Biomar S.A., 24231 Onzonilla (ES)
(72) Inventor: KASHMAN, Yoel, Pharma Mar S.A., 28760 Tres Cantos (ES); RUDI, Amira, Pharma Mar S.A., 28760 Tres Cantos (ES); YOSIEF, Tesfamariam, Pharma Mar S.A., 28760Tres Can0tos (ES); GRAVALOS, Dolores G., Pharma Mar S.A., 28760 Tres Cantos (ES)
(74) Representative: Ruffles, Graham Keith
(86) International application number: PCT/GB1998/003884
(87) International publication number: WO 1999/033832

(56) References cited:
- YOSIEF T ET AL: "Asmarines A-C;three novel cytotoxic metabolites from the marine sponge Raspailia sp." TETRAHEDRON LETT. (TELEAY,00404039);1998; VOL.39 (20); PP.3323-3326, XP002096056 Tel Aviv University;School of Chemistry; Ramat Aviv; 69978; Israel (IL)

## Description

The present invention relates to new cytotoxic alkaloids, Asmarine A and B, isolated from the sponge *Raspailia sp*.

### Background of the Invention

Marine organisms, especially soft corals, sponges and tunicates, provide many secondary metabolites and exhibit a varying degree of biological activity (Reference 1). A family of these metabolites is the diterpene-alkaloid family; in 1984 (Reference 2) it was reported the structure of four Agelasines:

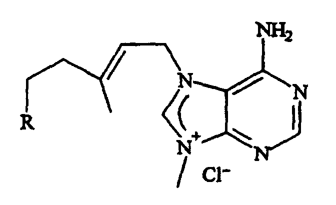

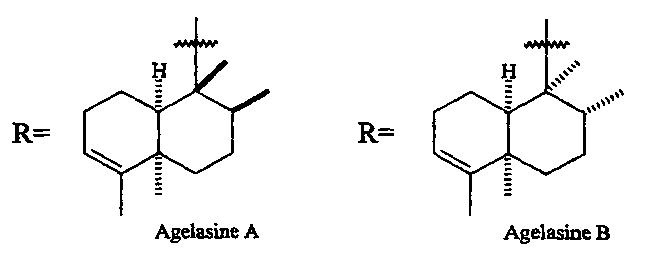

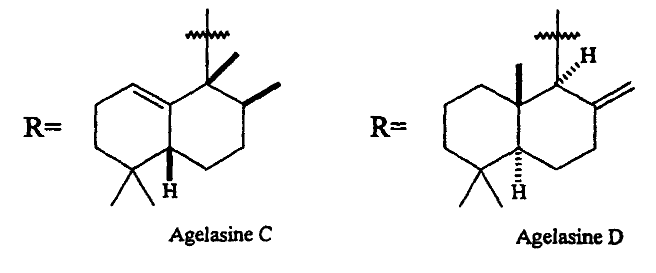

We have isolated from the sponge *Raspailia sp.* new cytotoxic diterpene-alkaloids related to this agelasine family.

Asmarines A, B and C are disclosed in Tetrahedron Lett., 1998, 39 (20), 3323-6, which was published after the earliest priority date of the present application.

### Summary of the Invention

The present invention provides new diterpene-alkaloid having either the formula (I) or the formula (II): 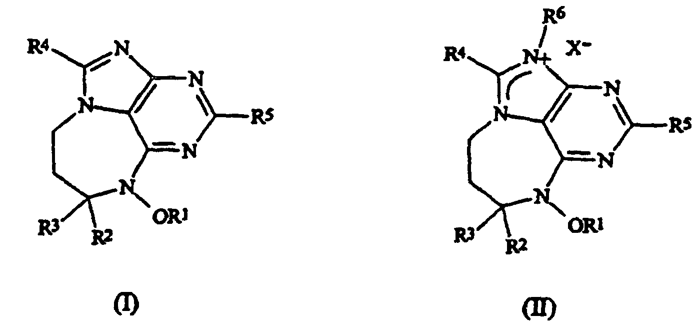 wherein R¹ represents hydrogen or lower alkyl or lower alkanoyl; R² represents hydrogen or lower alkyl; R³ is an alkyl group containing one or more isoprene units; R⁴ and R⁵ represent hydrogen or lower alkyl; R⁶ represent lower alkyl; X represents F or Cl or Br or I.

In the definitions of the groups in formulas (I) and (II), the lower alkyl and the lower alkyl moiety of the lower alkanoyl mean a straight-chain or branched alkyl group having 1 to 6 carbon atoms such as methyl, ethyl, propyl, isopropyl, butyl, isobutyl, sec-butyl, tert-butyl, pentyl, neopentyl and hexyl.

More particularly, the present invention relates to Asmarine A and Asmarine B, extracted and isolated from the sponge *Raspailia sp*. The structures of Asmarine A is the following:

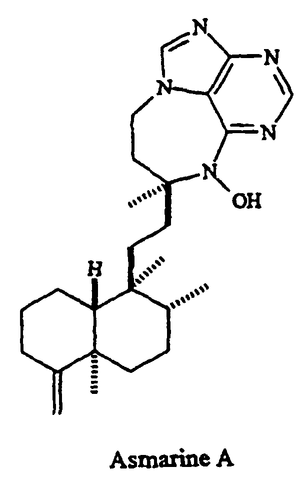

The stereochemistry shown is for a relative configuration.

Asmarine A and Asmarine B exhibit antitumor activity. In particular, Asmarine A and Asmarine B exhibit antitumor activity against cell lines derived from human solid tumors, such as human lung carcinoma, human colon carcinoma and human melanoma, and, the like, it is active against other tumor cell lines, like leukaemia and lymphoma.

The present invention also provides the use of a compound with either the formula (I) or the formula (II), in the manufacture of a medicament for the treatment of tumors. Such a medicament may optionally contain one or more antitumoral compounds.

The present invention further provides pharmaceutical compositions which contain as active ingredient a compound with either the formula (I) or the formula (II), as well as a process for its preparation.

Examples of pharmaceutical compositions include any solid (tablets, pills, capsules, granules, etc.) or liquid (solutions, suspensions or emulsions) with suitable formulation of oral, topical or parenteral administration, and they may contain the pure compound or in combination with any carrier or other pharmacologically active compounds. These compositions may need to be sterile when administered parenterally.

The correct dosage of a pharmaceutical composition comprising compounds with either the formula (I) or the formula (II), will vary according to the pharmaceutical formulation, the mode of application, and the particular situs, host and tumor being treated. Other factors like age, body weight, sex, diet, time of administration, rate of excretion, condition of the host, drug combinations, reaction sensitivities and severity of the disease shall be taken into account. Administration can be carried out continuously or periodically within the maximum tolerated dose.

### Antitumour Activity

Cells were maintained in logarithmic phase of growth in Eagle's Minimum Essential Medium, with Earle's Balanced Salts, with 2.0 mM L-glutamine, with non-essential amino acids, without sodium bicarbonate (EMEM/neaa); supplemented with 10% Fetal Calf Serum (FCS), 10⁻² M sodium bicarbonate and 0,1 g/l penicillin-G + streptomycin sulfate.

A screening procedure has been carried out to determine and compare the antitumor activity of these compounds, using an adapted form of the method described by Bergeron et al. (Reference 3). The antitumor cells employed were P-388 (suspension culture of a lymphoid neoplasm from DBA/2 mouse), A-549 (monolayer culture of a human lung carcinoma), HT-29 (monolayer culture of a human colon carcinoma) and MEL-28 (monolayer culture of a human melanoma).

P-388 cells were seeded into 16 mm wells at 1x10⁴ cells per well in 1 ml aliquots of MEM 5FCS containing the indicated concentration of drug. A separate set of cultures without drug was seeded as control growth to ensure that cells remained in exponential phase of growth. All determinations were carried out in duplicate. After three days of incubation at 37°C, 10% CO₂ in a 98% humid atmosphere, an approximately IC₅₀ was determined by comparing the growth in wells with drug to the growth in wells control.

A-549, HT-29 and MEL-28 cells were seeded into 16 mm wells at 2x10⁴ cells per well in 1 ml aliquots of MEM 10FCS containing the indicated concentration of drug. A separate set of cultures without drug was seeded as control growth to ensure that cells remained in exponential phase of growth. All determinations were carried out in duplicate. After three days of incubation at 37°C, 10% CO₂ in a 98% humid atmosphere, the wells were stained with 0.1% Crystal Violet. An approximately IC₅₀ was determined by comparing the growth in wells with drug to the growth in wells control.

The results of the *in vitro* cytotoxic assays for Asmarine A and Asmarine B with the cell lines P-388, A-549, HT-29 and MEL-28 are given in the following table:

| | IC₅₀ (µM) | | | |
|---|---|---|---|---|
| | **P-388** | **A-549** | **HT-29** | **MEL-28** |
| **Asmarine A** | 1.18 | 1.18 | 1.18 | 1.18 |
| | | | | |
| **Asmarine B** | 0.24 | 0.12 | 0.12 | 0.24 |

### Extraction and Isolation

Low-resolution mass spectra were recorded on a EIMS mass spectrometer. ¹H and ¹³C-NMR spectra were recorded on a Bruker ARX-500 spectrometer. All chemical shifts are reported with respect to TMS (δ=0 ppm).

*Raspailia sp.* (Rob Van Soest), (Class Demorspongia, Order poecilosclerida, Family Raspailiidea) was collected in Dahlak Archipelago, Eritrea by SCUBA diving to a depth of 23.5 m in May 1997. A reference sample is deposited in Tel Aviv University (ET-338). The sponge was frozen on site. The freeze dried sponge (20 gr) was extracted with ethyl acetate 2x to give a brown gum 1.2 g after evaporation. This brown gum was fractionated by Kupchon into four fractions: hexane, carbon tetrachloride, chloroform and water. The CHCl₃ and CCl₄ fractions were similar. These two fractions were combined and chromatographed on a sephadex LH-20 column eluting with MeOH:CHCl₃ (1:1) giving five fractions. Fractions (3-5) were further chromatographed repeatedly on a sephadex LH-20 with the same solvent system yielding Asmarine A (100 mg) as a solid, mp= 232 °C, m/z+ = 423 (C₂₅H₃₇N₅O), (100%) 188 (C₈H₆N₅O⁺), [α]_{D}²⁰ +55° (c=0.5, CHCl₃), IR 3400, 2928, 1600, 1553, 1451, 1400, 1388, 900 cm⁻¹, the structure of this Asmarine A was confirmed by X-ray analysis. With the same solvent was also isolated Asmarine B (120 mg) as an oil, m/z⁺ = 423 (C₂₅H₃₇N₅O), (100%) 188 (C₈H₆N₅O⁺), [α]_{D}²⁰.+60° (c= 0.5, CHCl₃), IR 3400, 2927, 1606, 1553, 1451, 1404, 1388, 900 cm⁻¹.

For NMR data of Asmarine A and B see next Table: in all cases the solvent is CDCl₃ and all chemical shifts are reported with respect to TMS (δ=0 ppm).

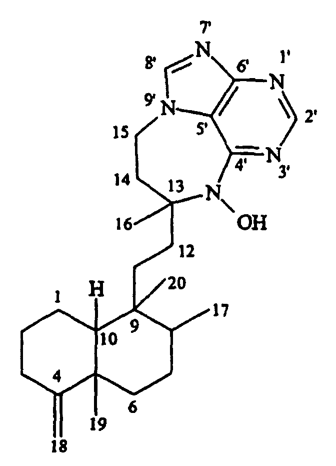

| | **δ**_{**C**} | | **δ**_{**H**} | |
|---|---|---|---|---|
| **C No.** | **Asmarin A** | **Asmarine B** | **Asmarine A** | **Asmarine B** |
| **1** | 21.82 t | 21.17 t | 1.70 d, 1H | 1.82 m,2 H |
| | | | 1.45 m, 1H | |
| **2** | 28.56 t | 24.13 t | 1.85 brd, 1H | 1.75 m, 1H |
| | | | 1.21 m, 1H | 1.60 m, 1H |
| **3** | 33.20 t | 31.65 t | 2.25 dt, 1H | 2.45 m, 1H |
| | | | 2.05 dd, 1H | 2.12 m, 1H |
| **4** | 160.59 s | 153.65 s | | |
| **5** | 40.05 s | 39.35 s | | |
| **6** | 37.24 t | 38.12 t | 1.50 m, 2H | 2.10 t, 1H |
| | | | | 1.20 t, 1H |
| **7** | 27.36 t | 27.22 t | 1.45 m, 2H | 1.54 m, 1H |
| | | | | 1.20 m, 1H |
| **8** | 36.68 d | 38.12 d | 1.37 m, 1H | 1.35 m, 1H |
| **9** | 39.26 s | 40.54 s | | |
| **10** | 48.62 d | 46.56 d | 1.05 d, 1H | 1.39 m, 1H, |
| **11** | 31.23 t | 31.05 t | 1.55 dt, 1H | 1.59 m, 1H |
| | | | 1.25 m, 1H | 1.30 t, 1H |
| **12** | 33.01 t | 31.55 t | 1.95 dt, 1H | 1.95 dt, 1H |
| | | | 1.43 m, 1H | 1.55 m, 1H |
| **13** | 64.20 s | 64.95 s | | |
| **14** | 36.68 t | 36.40 t | 2.50 dt, 1H | 2.55 m, 1H |
| | | | 2.15 dd, 1H | 2.25 m, 1H |
| **15** | 42.30 t | 42.33 t | 4.25 dt, 1H | 4.30 t, 2H |
| | | | 4.20 dd, 1H | |
| **16** | 21.75 q | 23.09 q | 1.44 s, 3H | 1.49 s, 3H |
| **17** | 15.93 q | 15.84 q | 0.70 d, 3H | 0.74 d, 3H |
| **18** | 102.45 t | 105.69 t | 4.60 s, 2H | 4.70 d, 2H |
| **19** | 20.08 q | 32.87 q | 1.00 s, 3H | 1.11 s, 3H |
| **20** | 18.28 q | 19.85 q | 0.65 s, 3H | 0.82 s, 3H |
| **2'** | 151.68 d | 151.64 d | 8.50 s, 1H | 8.50 d, 1H |
| **4'** | 149.00 s | 149.57 s | | |
| **5'** | 109.31 s | 109.32 s | | |
| **6'** | 158.70 s | 158.38 s | | |
| **8'** | 143.10 d | 143.32 d | 7.95 s, 1H | 7.95 s, 1H |

### References

1. Faulkner, D. *Nat.Prod.Rep.* **1997,** *14*, 259-302 and references therein.
2. Nakamura, H. et al. *Tetrahedron Lett.* **1984,** *25*, 2989-2992
3. Raymond J. Bergeron, Paul F. Cavanaugh, Jr., Steven J. Kline, Robert G. Hughes, Jr., Gary T. Elliot and Carl W. Porter. Antineoplastic and antiherpetic activity of spermidine catecholamide iron chelators. *Biochem. Bioph. Res. Comm.* **1984**, *121*, 848-854.

## Claims

1. A compound having the either the formula (I) or the formula (II): 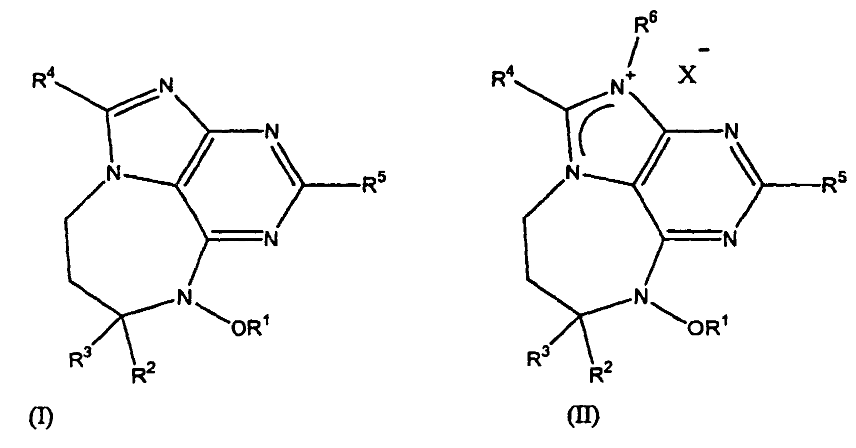 wherein R¹ represents hydrogen or lower alkyl or lower alkanoyl; R² represents hydrogen or lower alkyl; R³ is an alkyl group containing one or more isoprene units; R⁴ and R⁵ represent hydrogen or lower alkyl; R⁶ represent lower alkyl; X represents F or Cl or Br or I; and
the lower alkyl and the lower alkyl moiety of the lower alkanoyl mean a straight-chain or branched alkyl group having 1 to 6 carbon atoms such as methyl, ethyl, propyl, isopropyl, butyl, isobutyl, sec-butyl, tert-butyl, pentyl, neopentyl and hexyl.

2. A compound, Asmarine A, according to claim 1, having the following formula:

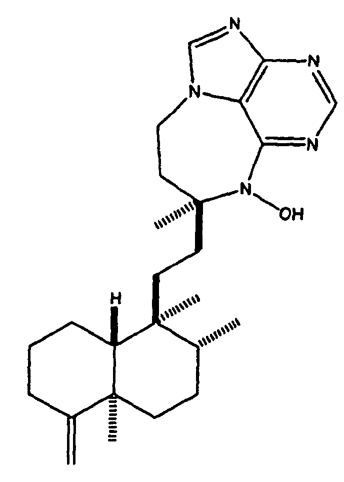

3. A compound, Asmarine B, according to claim 1, having the formula: 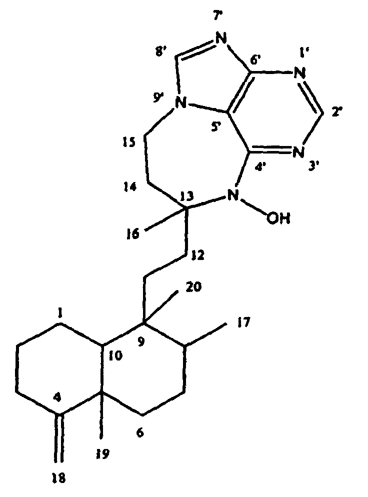 and having the following NMR data in CDCl₃:
| | δ_{C} | δ_{H} |
|---|---|---|
| C No. | Asmarine B | Asmarine B |
| 1 | 21.17 t | 1.82 m, 2H |
| 2 | 24.13 t | 1.75 m, 1H |
| | | 1.60 m, 1H |
| 3 | 31.65 t | 2.45 m, 1H |
| | | 2.12 m, 1H |
| 4 | 153.65 s | |
| 5 | 39.35 s | |
| 6 | 38.12 t | 2.10 t, 1H |
| | | 1.20 t, 1H |
| 7 | 27.22 t | 1.54 m, 1H |
| | | 1.20 m, 1H |
| 8 | 38.12 d | 1.35 m, 1H |
| 9 | 40.54 s | |
| 10 | 46.56 d | 1.39 m, 1H |
| 11 | 31.05 t | 1.59 m, 1H |
| | | 1.30 t, 1H |
| 12 | 31.55 t | 1.95 dt, 1H |
| | | 1.55 m, 1H |
| 13 | 64.95 s | |
| 14 | 36.40 t | 2.55 m, 1H |
| | | 2.25 m, 1H |
| 15 | 42.33 t | 4.30 t, 2H |
| 16 | 23.09 q | 1.49 s, 3H |
| 17 | 15.84q | 0.74 d, 3H |
| 18 | 105.69 t | 4.70 d, 2H |
| 19 | 32.87 q | 1.11 s, 3H |
| 20 | 19.85 q | 0.82 s, 3H |
| 2' | 151.64 d | 8.50 d, 1H |
| 4' | 149.57 s | |
| 5' | 109.32 s | |
| 6' | 158.38 s | |
| 8' | 143.32 d | 7.95 s, 1H |

4. Use of a compound with either the formula (I) or the formula (II), as defined in claim 1, in the manufacture of a medicament for the treatment of malignant tumors.

5. Use of Asmarine A (as defined in claim 2) or Asmarine B (as defined in claim 3), in the manufacture of a medicament for the treatment of malignant tumors.

6. A pharmaceutical preparation which contains as active ingredient a compound with either the formula (I) or the formula (II), as defined in claim 1.

7. A pharmaceutical preparation which contains as active ingredient Asmarine A (as defined in claim 2) or Asmarine B (as defined in claim 3).

8. Use of a compound with either the formula (I) or the formula (II), as defined in claim 1, together with one or more other antitumoral compounds, in the manufacture of a medicament for the treatment of malignant tumors.

9. Use of Asmarine A (as defined in claim 2) or Asmarine B (as defined in claim 3), together with one or more other antitumoral compounds, in the manufacture of a medicament for the treatment of malignant tumors.

## Patentansprüche

1. Verbindung, welche entweder durch die Formel (I) oder durch die Formel (II) dargestellt wird: 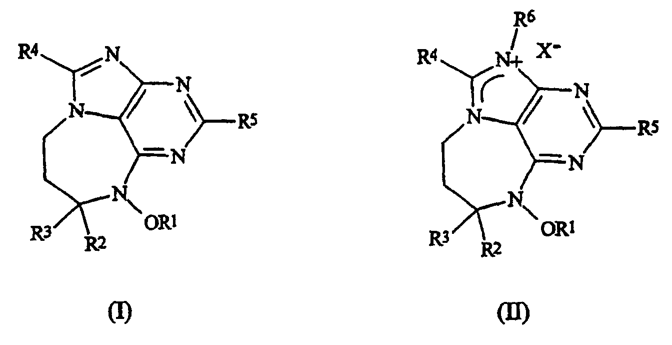 wobei:
R¹ für Wasserstoff oder ein niedrigeres Alkyl oder ein niedrigeres Alkanoyl steht; R² für Wasserstoff oder ein niedrigeres Alkyl steht; R³ eine Alkylgruppe darstellt, welche eine oder mehrere Isopreneinheiten enthält; R⁴ und R⁵ für Wasserstoff oder ein niedrigeres Alkyl stehen; R⁶ ein niedrigeres Alkyl darstellt; X für F oder Cl oder Br oder I steht; und
das niedrigere Alkyl und die niedrigere Alkylhälfte des niedrigeren Alkanoyls eine geradlinige Kette oder eine verzweigte Alkylgruppe bedeutet, welche 1 bis 6 Kohlenstoffatomen enthält, wie etwa Methyl, Ethyl, Propyl, Isopropyl, Butyl, Isobutyl, sec-Butyl, tert-Butyl, Pentyl, Neopentyl und Hexyl.

2. Verbindung, Asmarin A, gemäß Anspruch 1, mit der folgenden Formel:

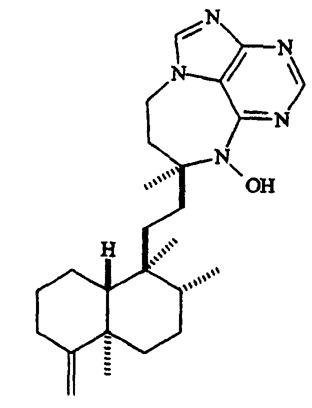

3. Verbindung, Asmarin B, gemäß Anspruch 1, mit der Formel: 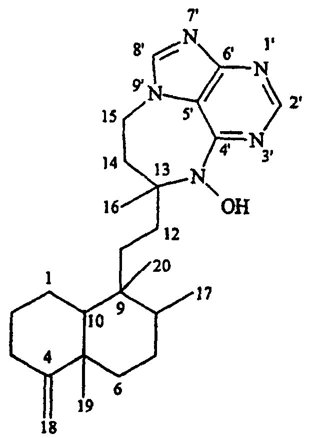 und mit den folgenden NMR Daten in CDCl₃:
| | δ_{C} | δ_{H} |
|---|---|---|
| C No. | Asmarin B | Asmarin B |
| 1 | 21,17 t | 1,82m, 2H |
| 2 | 24,13 t | 1,75 m, 1H |
| | | 1,60 m, 1H |
| 3 | 31,65 t | 2,45 m, 1H |
| | | 2,12 m, 1H |
| 4 | 153,65 s | |
| 5 | 39,35 s | |
| 6 | 38,12 t | 2,10 t, 1H |
| | | 1,20 t, 1H |
| 7 | 27,22 t | 1,54 m, 1H |
| | | 1,20 m, 1H |
| 8 | 38,12 d | 1,35 m, 1H |
| 9 | 40,54 s | |
| 10 | 46,56 d | 1,39 m, 1H |
| 11 | 31,05 t | 1,59 m, 1H |
| | | 1,30 t, 1H |
| 12 | 31,55 t | 1,95 dt, 1H |
| | | 1,55 m, 1H |
| 13 | 64,95 s | |
| 14 | 36,40 t | 2,55 m, 1H |
| | | 2,25 m, 1H |
| 15 | 42,33 t | 4,30 t, 2H |
| 16 | 23,09 q | 1,49 s, 3H |
| 17 | 15,84 q | 0,74 d, 3H |
| 18 | 105,69 t | 4,70 d, 2H |
| 19 | 32,87 q | 1,11 s, 3H |
| 20 | 19,85 q | 0,82 s, 3H |
| 2' | 151,64 d | 8,50 d, 1H |
| 4' | 149,57 s | |
| 5' | 109,32 s | |
| 6' | 158,38 s | |
| 8' | 143,32 d | 7,95 s, 1H |

4. Verwendung einer Verbindung, welche entweder durch die Formel (I) oder durch die Formel (II) dargestellt wird, so wie in Anspruch 1 definiert, bei der Herstellung eines Medikamentes zur Behandlung von bösartigen Tumoren.

5. Verwendung von Asmarin A (so wie in Anspruch 2 definiert) oder von Asmarin B (so wie in Anspruch 3 definiert) bei der Herstellung eines Medikamentes zur Behandlung von bösartigen Tumoren.

6. Pharmazeutisches Arzneimittel, welches als ein aktives Ingrediens eine Verbindung enthält, welche entweder durch die Formel (I) oder durch die Formel (II) dargestellt wird, so wie in Anspruch 1 definiert.

7. Pharmazeutisches Arzneimittel, welches als ein aktives Ingrediens Asmarin A (so wie in Anspruch 2 definiert) oder Asmarin B (so wie in Anspruch 3 definiert) enthält.

8. Verwendung einer Verbindung, welche entweder durch die Formel (I) oder durch die Formel (II) dargestellt wird, so wie in Anspruch 1 definiert, zusammen mit einer oder mit mehreren anderen antitumoralen Verbindungen bei der Herstellung eines Medikamentes zur Behandlung von bösartigen Tumoren.

9. Verwendung von Asmarin A (so wie in Anspruch 2 definiert) oder von Asmarin B (so wie in Anspruch 3 definiert) zusammen mit einer oder mit mehreren anderen antitumoralen Verbindungen bei der Herstellung eines Medikamentes zur Behandlung von bösartigen Tumoren.

## Revendications

1. Composé présentant soit la formule (I), soit la formule (II): 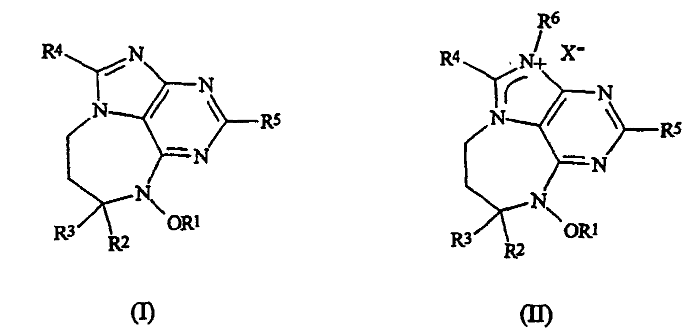 dans lesquelles,
R¹ représente un hydrogène ou un groupe alkyle inférieur ou un groupe alcanoyle inférieur; R² représente un hydrogène ou un groupe alkyle inférieur; R³ est un groupe alkyle contenant une ou plusieurs unités d'isoprène; R⁴ et R⁵ représentent un hydrogène ou un groupe alkyle inférieur; R⁶ représente un groupe alkyle inférieur; X représente F ou Cl ou Br ou I; et
le groupe alkyle inférieur et la fraction d'alkyle inférieur du groupe alcanoyle inférieur signifient un groupe alkyle à chaîne droite ou ramifié possédant de 1 à 6 atomes de carbone, tel qu'un groupe méthyle, éthyle, propyle, isopropyle, butyle, isobutyle, sec-butyle, tert-butyle, pentyle, néopentyle et hexyle.

2. Composé, Asmarine A, suivant la revendication 1, présentant la formule suivante:

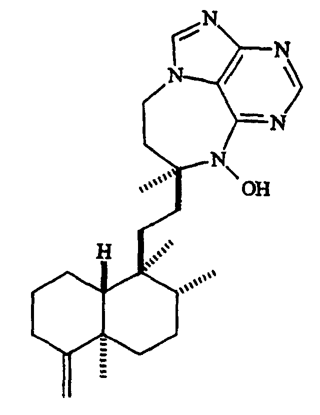

3. Composé, Asmarine B, suivant la revendication 1, présentant la formule: 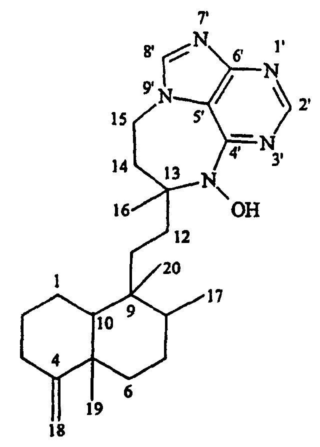 et avec les données RMN suivantes dans CDCl₃:
| | δ_{C} | δ_{H} |
|---|---|---|
| C No. | Asmarine B | Asmarine B |
| 1 | 21,17 t | 1,82m, 2H |
| 2 | 24,13 t | 1,75 m, 1H |
| | | 1,60 m, 1H |
| 3 | 31,65 t | 2,45 m, 1H |
| | | 2,12 m, 1H |
| 4 | 153,65 s | |
| 5 | 39,35 s | |
| 6 | 38,12 t | 2,10 t, 1H |
| | | 1,20 t, 1H |
| 7 | 27,22 t | 1,54 m, 1H |
| | | 1,20 m, 1H |
| 8 | 38,12 d | 1,35 m, 1H |
| 9 | 40,54 s | |
| 10 | 46,56 d | 1,39 m, 1H |
| 11 | 31,05 t | 1,59 m, 1H |
| | | 1,30 t, 1H |
| 12 | 31,55 t | 1,95 dt, 1H |
| | | 1,55 m, 1H |
| 13 | 64,95 s | |
| 14 | 36,40 t | 2,55 m, 1H |
| | | 2,25 m, 1H |
| 15 | 42,33 t | 4,30 t, 2H |
| 16 | 23,09 q | 1,49 s, 3H |
| 17 | 15,84 q | 0,74 d, 3H |
| 18 | 105,69 t | 4,70 d, 2H |
| 19 | 32,87 q | 1,11 s, 3H |
| 20 | 19,85 q | 0,82 s, 3H |
| 2' | 151,64 d | 8,50 d, 1H |
| 4' | 149,57 s | |
| 5' | 109,32 s | |
| 6' | 158,38 s | |
| 8' | 143,32 d | 7,95 s, 1H |

4. Utilisation d'un composé avec soit la formule (I), soit la formule (II), telles que définies dans la revendication 1, dans la fabrication d'un médicament pour le traitement de tumeurs malignes.

5. Utilisation d'Asmarine A (telle que définie dans la revendication 2) ou d'Asmarine B (telle que définie dans la revendication 3), dans la fabrication d'un médicament pour le traitement de tumeurs malignes.

6. Préparation pharmaceutique qui contient en tant que principe actif un composé avec soit la formule (I), soit la formule (II), telles que définies dans la revendication 1.

7. Préparation pharmaceutique qui contient en tant que principe actif l'Asmarine A (telle que définie dans la revendication 2) ou l'Asmarine B (telle que définie dans la revendication 3).

8. Utilisation d'un composé avec soit la formule (I), soit la formule (II), telles que définies dans la revendication 1, accompagné d'un ou de plusieurs autres composés anti-tumoraux, dans la fabrication d'un médicament pour le traitement de tumeurs malignes.

9. Utilisation d'Asmarine A (telle que définie dans la revendication 2) ou d'Asmarine B (telle que définie dans la revendication 3), accompagnée d'un ou de plusieurs autres composés anti-tumoraux, dans la fabrication d'un médicament pour le traitement de tumeurs malignes.
